# EUROPEAN PATENT APPLICATION

(11) **EP 1 632 228 A1**
(43) Date of publication of application: **08.03.2006**
(21) Application number: 04736116.7
(22) Date of filing: 04.06.2004
(51) Int. Cl.: A61K 31/4196, A61P 31/10

(54) **MEDICINAL COMPOSITION CONTAINING TRIAZOLE COMPOUND**

(30) Priority: 06.06.2003 JP 2003161945
(71) Applicant: Sankyo Company, Limited, Tokyo 103-8426 (JP)
(72) Inventor: YADA, Shuichi C/o Sankyo Company, Limited, Shinagawa-ku, Tokyo 1408710 (JP); OH-UCHI, Yuko C/o Sankyo Company, Limited, Shinagawa-ku, Tokyo 1408710 (JP)
(74) Representative: Gibson, Christian John Robert
(86) International application number: PCT/JP2004/008170
(87) International publication number: WO 2004/108134

(57) **Abstract**

A medicinal composition comprising a triazole compound having the general formula (I): 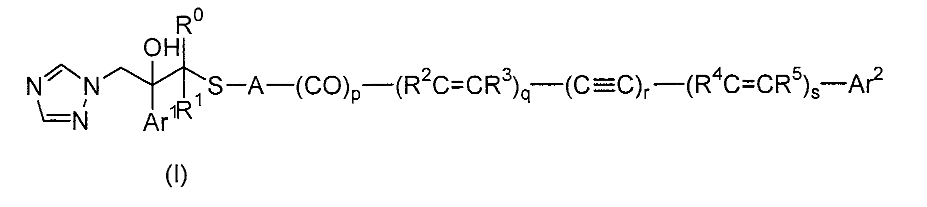 (wherein Ar¹ is a phenyl group which may be substituted; Ar² is a phenyl which may be substituted; R⁰ is a hydrogen atom; R¹ is a lower alkyl group; R², R³, R⁴ and R⁵ are the same or different and each is a hydrogen atom or a lower alkyl group; p is 0 or 1; q, r and s are each 0, 1 or 2; and A is 1,3-dioxane), or a pharmaceutically acceptable ester thereof or a pharmaceutically acceptable salt thereof, and a pharmaceutically acceptable basic substance.

## Description

### Technical Field

The present invention relates to a stabilized medicinal composition comprising a triazole compound having a specific chemical structure or a pharmaceutically acceptable ester thereof or a pharmaceutically acceptable salt thereof, and a pharmaceutically acceptable basic substance.

### Background Art

Various triazole compounds are known in the prior art as agents for treatment of fungal infections. For example, triazole compounds having a tertiary hydroxyl group are described in Japanese Patent No. 2902345 and Japanese Patent No. 3240129. However, although the above-mentioned patent references disclose medicinal compositions comprising a triazole compounds as agents for treatment of fungal infections, addition of an antioxidant as a stabilizer is only described in Japanese Patent No. 3240129 and preservation stability and the like by blending with a basic substance are not described.

### Disclosure of the Invention

The present inventors have conducted intensive studies on a medicinal composition comprising a triazole compound which has a specific chemical structure, and found that a medicinal composition having basic substances blended with a triazole compound has an excellent preservation and handling stability (particularly preservation stability) and it is useful as a medicinal preparation [particularly, as a therapeutic agent and a prophylactic agent (preferably a therapeutic agent) for fungal diseases] for warm-blooded animals (particularly for human beings), and completed the present invention.

The present invention is a stabilized medicinal composition comprising a triazole compound having the general formula (I): 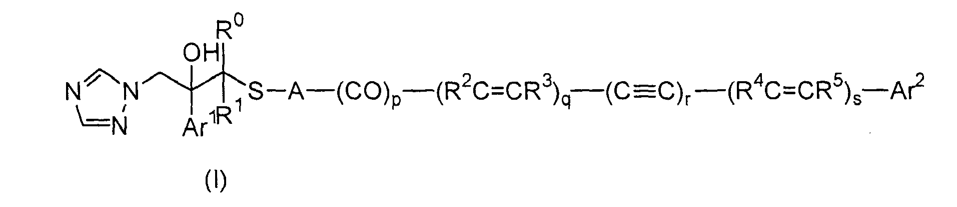 [wherein Ar¹ represents a phenyl group or a phenyl group having from 1 to 3 substituents (wherein each of the substituents represents a halogen atom or a trifluoromethyl group);
Ar² represents a phenyl group, a 5- or 6-membered aromatic heterocyclic group (wherein the aromatic heterocyclic group has at least one nitrogen, oxygen or sulfur atom), or a phenyl or 5- or 6-membered aromatic heterocyclic group having from 1 to 3 substituents {wherein each of the substituents represents a lower alkyl group, a lower alkoxy group, a halogen atom, a lower alkyl group substituted with a halogen atom, a lower alkoxy group substituted with a halogen atom, a nitro group, a cyano group, an -S(O)ₘR⁶ group (wherein R⁶ represents a lower alkyl group which may be substituted with a halogen atom and m represents 0, 1 or 2) or an NHCOR⁷ group (wherein R⁷ represents a lower alkyl group) and the aromatic heterocyclic group has at least one nitrogen, oxygen or sulfur atom};
R⁰ represents a hydrogen atom or a lower alkyl group;
R¹ represents a lower alkyl group;
R², R³, R⁴ and R⁵ are the same or different and each represents a hydrogen atom, a lower alkyl group or a lower alkyl group substituted with a halogen atom and R², R³, R⁴ and R⁵ each independently represents the same or different group when q and/or s represents 2;
p represents 0 or 1;
q, r and s each represent 0, 1 or 2; and
A represents 1,3-dioxane],
or a pharmaceutically acceptable ester thereof, or a pharmaceutically acceptable salt thereof, and
a pharmaceutically acceptable basic substance.

The above-mentioned halogen atom is, for example, a fluorine, chlorine or bromine atom, and preferably is a fluorine or chlorine atom.

The lower alkyl group is, for example, a methyl, ethyl, propyl, isopropyl, butyl, isobutyl, sec-butyl or tert-butyl group, and preferably is a methyl, ethyl, propyl or isopropyl group.

The lower alkoxy group is, for example, a methoxy, ethoxy, propoxy, isopropoxy, butoxy, isobutoxy, sec-butoxy or tert-butoxy group, and preferably is a methoxy, ethoxy, propoxy or isopropoxy group.

The 5- or 6-membered aromatic heterocyclic group of Ar² is, for example, a furyl, thienyl, pyrrolyl, pyrazolyl, imidazolyl, oxazolyl, thiazolyl, pyridyl, pyrimidyl or pyrazyl group, and preferably is a furyl, thienyl, pyrrolyl or pyridyl group.

The 1,3-dioxane of A is, for example, 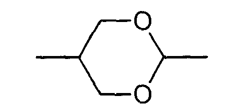 or 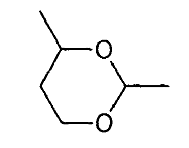 and preferably is

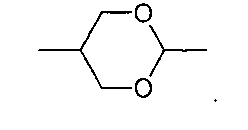

A preferred triazole compound (I) is
a compound wherein Ar¹ is a dichlorophenyl, difluorophenyl, chlorophenyl, fluorophenyl, (trifluoromethyl)phenyl or fluoro(trifluoromethyl)phenyl group, and preferably is a 2,4-dichlorophenyl, 2,4-difluorophenyl, 4-chlorophenyl, 4-fluorophenyl, 4-(trifluoromethyl)phenyl or 2-fluoro-4-(trifluoromethyl)phenyl group, and particularly preferably is a 2,4-dichlorophenyl, 2,4-difluorophenyl or 4-(trifluoromethyl)phenyl group;
a compound wherein Ar² is a fluorophenyl group, chlorophenyl group, difluorophenyl group, dichlorophenyl group, (trifluoromethyl)phenyl group, (trichloromethyl)phenyl group, fluoro(trifluoromethyl)phenyl group, (difluoromethoxy)phenyl group, (trifluoromethoxy)phenyl group, (2,2,2-trifluoroethoxy)phenyl group, (1,1,2,2-tetrafluoroethoxy)phenyl group, (2,2,3,3-tetrafluoropropoxy)phenyl group, fluoro(2,2,3,3-tetrafluoropropoxy)phenyl group, nitrophenyl group, fluoronitrophenyl group, cyanophenyl group, cyano-fluorophenyl group, chlorocyanophenyl group, (methylthio)phenyl group, (methylsulfinyl)phenyl group, (methylsulfonyl)phenyl group, (trifluoromethylthio)phenyl group, (trifluoromethylsulfinyl)phenyl group, (trifluoromethylsulfonyl)phenyl group, chloropyridyl group, (trifluoromethyl)pyridyl group, (2,2,3,3-tetrafluoropropoxy)pyridyl group, (trifluoromethyl)furyl group, chlorothienyl group or (trifluoromethyl)thienyl group, and preferably is a 4-fluorophenyl group, 4-chlorophenyl group, 2,4-difluorophenyl group, 2,4-dichlorophenyl group, 4-(trifluoromethyl)phenyl group, 4-(trichloromethyl)phenyl group, 2-fluoro-4-(trifluoromethyl)phenyl group, 4-(difluoromethoxy)phenyl group, 3-(trifluoromethoxy)phenyl group, 4-(trifluoromethoxy)phenyl group, 4-(2,2,2-trifluoroethoxy)phenyl group, 4-(1,1,2,2-tetrafluoroethoxy)phenyl group, 4-(2,2,3,3-tetrafluoropropoxy)phenyl group, 2-fluoro-4-(2,2,3,3-tetrafluoropropoxy)phenyl group, 4-nitrophenyl group, 2-fluoro-4-nitrophenyl group, 4-cyanophenyl group, 4-cyano-2-fluorophenyl group, 4-cyano-3-fluorophenyl group, 2-chloro-4-cyanophenyl group, 4-(methylthio)phenyl group, 4-(methylsulfinyl)phenyl group, 4-(methylsulfonyl)phenyl group, 4-(trifluoromethylthio)phenyl group, 4-(trifluoromethylsulfinyl)phenyl group, 4-(trifluoromethylsulfonyl)phenyl group, 6-chloro-3-pyridyl group, 6-(trifluoromethyl)-3-pyridyl group, 5-chloro-2-pyridyl group, 6-(2,2,3,3-tetrafluoropropoxy)-3-pyridyl group, 5-(trifluoromethyl)-2-furyl group, 5-chloro-2-thienyl group or 5-(trifluoromethyl)-2-thienyl group, and particularly preferably is a 4-chlorophenyl group, 4-cyano-2-fluorophenyl group, 4-cyano-3-fluorophenyl group, 4-(trifluoromethylthio)phenyl group, 4-(trifluoromethylsulfonyl)phenyl group, 4-(trifluoromethyl)phenyl group, 4-(trifluoromethoxy)phenyl group, 4-(1,1,2,2-tetrafluoroethoxy)phenyl group or 4-(2,2,3,3-tetrafluoropropoxy)phenyl group, and most preferably is 4-cyano-2-fluorophenyl;
a compound wherein R⁰ is a hydrogen atom, methyl group, ethyl group or propyl group, and preferably is a hydrogen atom, methyl group or ethyl group, and particularly preferably is a hydrogen atom or a methyl group;
a compound wherein R¹ is a methyl, ethyl or propyl group, and preferably is a methyl or ethyl group and particularly preferably is a methyl group;
a compound wherein R², R³, R⁴ and R⁵ are the same or different and each is a hydrogen atom, methyl group, ethyl group, propyl group or trifluoromethyl group, and preferably is a hydrogen atom, a methyl or trifluoromethyl group and particularly preferably is a hydrogen atom or a trifluoromethyl group;
a compound wherein p is 0 or 1 and particularly preferably p is 0;
a compound wherein q is 0, 1 or 2 and particularly preferably q is 1;
a compound wherein r is 0, 1 or 2 and particularly preferably r is 0 or 1;
a compound wherein s is 0, 1 or 2 and particularly preferably s is 1.
Examples of preferred triazole compounds (I) are
2-(2,4-difluorophenyl)-3-[[2-[2-[4-(trifluoromethyl)phenyl]vinyl]-1,3-dioxan-5-yl]thio]-1-(1H-1,2,4-triazol-1-yl)-2-butanol,
2-(2,4-difluorophenyl)-1-(1H-1,2,4-triazol-1-yl)-3-[[2-[2-[4-(trifluoromethoxy)phenyl]vinyl]-1,3-dioxan-5-yl]thio]-2-butanol,
2-(2,4-difluorophenyl)-1-(1H-1,2,4-triazol-1-yl)-3-[[2-[4-[4-(trifluoromethyl)phenyl]-1,3-butadien-1-yl]-1,3-dioxan-5-yl]thio]-2-butanol,
2-(2,4-difluorophenyl)-1-(1H-1,2,4-triazol-1-yl)-3-[[2-[4-(4-cyano-2-fluorophenyl)-1,3-butadien-1-yl]-1,3-dioxan-5-yl] thio]-2-butanol,
2-(2,4-difluorophenyl)-3-[[2-[4-[4-(2,2,3,3-tetrafluoropropoxy)phenyl]-1,3-butadien-1-yl]-1,3-dioxan-5-yl]thio]-1-(1H-1,2,4-triazol-1-yl)-2-butanol,
2-(2,4-difluorophenyl)-3-[[2-[4-(4-chlorophenyl)-4,4,4-trifluoro-1,3-pentadien-1-yl]-1,3-dioxan-5-yl]thio]-1-(1H-1,2,4-triazol-1-yl)-2-butanol,
3-methyl-1-(1H-1,2,4-triazol-1-yl)-2-[4-(trifluoromethyl)phenyl]-3-[[2-[4-[(trifluoromethyl)phenyl]-1,3-butadien-1-yl]-1,3-dioxan-5-yl]thio]-2-butanol,
2-(2,4-difluorophenyl)-1-(1H-1,2,4-triazol-1-yl)-3-[[2-[4-[4-(trifluoromethylthio)phenyl]-1,3-butadien-1-yl]-1,3-dioxan-5-yl]thio]-2-butanol,
3-[[2-[4-[4-(2,2,3,3-tetrafluoropropoxy)phenyl]-1,3-butadien-1-yl]-1,3-dioxan-5-yl]thio]-1-(1H-1,2,4-triazol-1-yl)-2-[4-(trifluoromethyl)phenyl]-2-butanol,
1-(1H-1,2,4-triazol-1-yl)-2-[4-(trifluoromethyl)phenyl]-3-[[2-[4-[4-(trifluoromethyl)phenyl]-1,3-butadien-1-yl]-1,3-dioxan-5-yl]thio]-2-butanol,
2-(2,4-difluorophenyl)-1-(1H-1,2,4-triazol-1-yl)-3-[[2-[4-[4-(trifluoromethylsulfinyl)phenyl]-1,3-butadien-1-yl]-1,3-dioxan-5-yl]thio]-2-butanol,
2-(2,4-difluorophenyl)-1-(1H-1,2,4-triazol-1-yl)-3-[[2-[6-[4-(trifluoromethyl)phenyl]-1,3,5-hexatrien-1-yl]-1,3-dioxan-5-yl]thio]-2-butanol,
(2R,3R)-3-[[trans-2-[(1E,3E)-4-(4-cyano-2-fluorophenyl)-1,3-butadien-1-yl]-1,3-dioxan-5-yl] thio]-2-(2,4-difluorophenyl)-1-(1H-1,2,4-triazol-1-yl)-2-butanol,
2-(2,4-difluorophenyl)-3-methyl-1-(1H-1,2,4-triazol-1-yl)-3-[[2-[4-[4-(trifluoromethyl)phenyl]-1,3-butadien-1-yl]-1,3-dioxan-5-yl]thio]-2-butanol, and
2-(2,4-difluorophenyl)-1-(1H-1,2,4-triazol-1-yl)-3-[[2-[4-[4-(trifluoromethyl)phenyl]-1-buten-3-yn-1-yl]-1,3-dioxan-5-yl]thio]-2-butanol,
and more preferably is (2R,3R)-3-[[trans-2-[(lE,3E)-4-(4-cyano-2-fluorophenyl)-1,3-butadien-1-yl]-1,3-dioxan-5-yl]thio]-2-(2,4-difluorophenyl)-1-(1H-1,2,4-triazol-1-yl)-2-butanol.

The triazole compound (I), which is a constituent ingredient of the medicinal composition of the present invention, can be converted to a pharmaceutically acceptable ester by acylation according to a conventional method, and such esters are also included in the present invention. Such esters can be, for example, esters with a saturated or unsaturated C₁-C₁₀ aliphatic monocarboxylic acid such as formic acid, acetic acid, propionic acid, butyric acid, acrylic acid, crotonic acid and propiolic acid; esters with a saturated or unsaturated C₂-C₁₀ aliphatic dicarboxylic acid such as fumaric acid, maleic acid, oxalic acid, malonic acid and succinic acid; esters with a C₇-C₁₂ aromatic carboxylic acid which may be substituted with 1 to 3 substituents selected from the group consisting of a C₁-C₄ alkyl group, a C₁-C₄ alkoxy group and a halogen atom such as benzoic acid, methylbenzoic acid, methoxybenzoic acid, fluorobenzoic acid and chlorobenzoic acid; esters with a C₁₋C₁₀ sulfonic acid such as methanesulfonic acid, trifluoromethanesulfonic acid, ethanesulfonic acid, benzenesulfonic acid and toluenesulfonic acid; esters with an amino acid such as glutamic acid and aspartic acid; carbonic acid ester; esters with a carbonic acid mono(C₁-C₆ alkyl) ester such as carbonic acid monomethyl ester, carbonic acid monoethyl ester, carbonic acid monopropyl ester and carbonic acid monobutyl ester; esters with a carbonic acid mono(C₆-C₁₀ aromatic hydrocarbon) ester such as carbonic acid monophenyl ester; phosphoric acid esters; esters with a phosphoric acid mono- or di(C₁-C₆ alkyl) ester such as phosphoric acid monomethyl ester, phosphoric acid dimethyl ester, phosphoric acid monoethyl ester and phosphoric acid diethyl ester; or esters with a phosphoric acid mono- or di(C₆-C₁₀ aromatic hydrocarbon) ester such as phosphoric acid monophenyl ester, phosphoric acid diphenyl ester, and preferably esters with a C₁-C₁₀ aliphatic monocarboxylic acid, esters with a C₂-C₁₀ aliphatic dicarboxylic acid, or esters with a C₇-C₁₂ aromatic carboxylic acid which may be substituted with 1 to 3 substituents selected from the group consisting of a C₁-C₄ alkyl group, a C₁-C₄ alkoxy group and a halogen atom, and more preferably esters with a C₁-C₁₀ aliphatic monocarboxylic acid.

The triazole compound (I) or a pharmaceutically acceptable ester thereof which is a constituent ingredient of the medicinal composition of the present invention has a basic group, and can be converted to a pharmaceutically acceptable salt (preferably, a salt of the triazole compound (I)) by treating with an acid according to a conventional method, and such salts are also included in the present invention. Such salts can be, for example, halogeno hydro acid salts such as hydrofluoric acid salts, hydrochloric acid salts, hydrobromic acid salts and hydroiodic acid salts; nitrates; perchlorates; sulfates; phosphates; carbonates; C₁-C₆ alkyl sulfonic acid salts which may be substituted with a fluoro group such as methanesulfonic acid salts, trifluoromethanesulfonic acid salts, ethanesulfonic acid salts, pentafluoroethanesulfonic acid salts and propanesulfonic acid salts; C₆-C₁₀ arylsulfonic acid salts such as benzenesulfonic acid salts, p-toluenesulfonic acid salts, 2,4-dimethylbenzenesulfonic acid salts, 2,4,6-trimethylbenzenesulfonic acid salts, 4-ethylbenzenesulfonic acid salts, 1-naphthalenesulfonic acid salts and 2-naphthalenesulfonic acid salts; saturated or unsaturated C₁-C₁₀ aliphatic carboxylic acid salts which may be substituted with a fluorine atom such as acetic acid salts, trifluoroacetic acid salts, propionic acid salts, butyric acid salts, oxalic acid salts, malonic acid salts, fumaric acid salts, maleic acid salts, succinic acid salts, citric acid salts and tartaric acid salts; C₇-C₁₂ aromatic carboxylic acid salts such as benzoic acid salts and phthalic acid salts; or amino acid salts such as glutamic acid salts and aspartic acid salts; and are preferably hydrochloric acid salts, hydrobromic acid salts, nitrates, sulfates, phosphates, benzenesulfonic acid salts, p-toluenesulfonic acid salts, 2,4-dimethylbenzenesulfonic acid salts, 2,4,6-trimethylbenzenesulfonic acid salts, 4-ethylbenzenesulfonic acid salts, 2-naphthalenesulfonic acid salts, fumaric acid salts or phthalic acid salts, more preferably hydrochloric acid salts, sulfates, benzenesulfonic acid salts, p-toluenesulfonic acid salts, 2-naphthalenesulfonic acid salts, fumaric acid salts or phthalic acid salts and still more preferably, p-toluenesulfonic acid salts or fumaric acid salts and, most preferably, fumaric acid salts.

The triazole compound (I), which is a constituent ingredient of the medicinal composition of the present invention, can exist as a hydrate or solvate, and either of them or a mixture thereof are included in the present invention.

The triazole compound (I), which is a constituent ingredient of the medicinal composition of the present invention, has at least two asymmetric carbons, and enantiomers and diastereomers exist. Both of the enantiomers can be obtained by a standard technique for optical resolution, or a technique of asymmetric synthesis. The diastereomers can be separated by using standard separating methods such as fractional recrystallization and chromatography. The triazole compound (I) of the present invention includes one or a mixture of these isomers.

The basic substance which is a constituent ingredient of the medicinal composition of the present invention can be a common pharmaceutically acceptable basic compound, and as long as the pH shown by the solution or distributed liquid exceeds 7, there is no particular limitation. This basic substance may be a substance in a range from water-soluble substances to hardly water-soluble or substantially water-insoluble substances, and for example, an alkaline metal hydroxide such as lithium hydroxide, sodium hydroxide, and potassium hydroxide; an alkaline earth metal hydroxide such as magnesium hydroxide, calcium hydroxide and barium hydroxide; aluminum hydroxide; an alkaline metal carbonate such as lithium carbonate, sodium carbonate and potassium carbonate; an alkaline earth metal carbonate such as magnesium carbonate, calcium carbonate and barium carbonate; an alkaline metal bicarbonate such as lithium bicarbonate, sodium bicarbonate and potassium bicarbonate; an alkaline metal dihydrogen phosphate such as lithium dihydrogen phosphate, sodium dihydrogen phosphate and potassium dihydrogen phosphate; a dialkaline metal hydrogen phosphate such as dilithium hydrogen phosphate, disodium hydrogen phosphate and dipotassium hydrogen phosphate; a tri(alkaline-metal) phosphate such as trilithium phosphate, trisodium phosphate and tripotassium phosphate; an alkaline earth metal dihydrogen phosphate such as magnesium dihydrogen phosphate, calcium dihydrogen phosphate and barium dihydrogen phosphate; an alkaline earth metal monohydrogen phosphate such as magnesium hydrogen phosphate, calcium monohydrogen phosphate and barium hydrogen phosphate; an alkaline earth metal phosphate such as magnesium phosphate, calcium phosphate and barium phosphate; an alkaline earth metal oxide such as barium oxide, magnesium oxide and calcium oxide; aluminum oxide; an alkaline metal silicate such as lithium silicate, sodium silicate and potassium silicate; an alkaline earth metal silicate such as barium silicate, magnesium silicate and calcium silicate; a complex silicate-aluminum compound such as silicate-alumina; a complex aluminum-magnesium compound such as hydrotalcite, synthetic hydrotalcite, magnesium aluminosilicate, magnesium aluminometasilicate, aluminum magnesium silicate and magnesium alumina hydroxide; a basic amino acid such as L-arginine and L-lysine; a basic polymer such as aminoalkylmethacrylate copolymer; an amine such as monoethanolamine, diethanolamine, diisopropanolamine, triethanolamine, triisopropanolamine and ethylenediamine; or a mixture thereof, and preferably is an alkaline metal hydroxide, alkaline earth metal hydroxide, aluminum hydroxide, an alkaline metal carbonate, alkaline earth metal carbonate, alkaline metal bicarbonate, dialkaline metal hydrogen phosphate, tri(alkaline-metal) phosphate, alkaline earth metal monohydrogen phosphate, alkaline earth metal phosphate, alkaline earth metal oxide, alkaline earth metal silicate, complex aluminum-magnesium compound or a mixture thereof, and more preferably, an alkaline earth metal hydroxide, complex aluminum-magnesium compound or a mixture thereof. Specifically the basic substance which is a constituent ingredient of the medicinal composition of the present invention is preferably sodium hydroxide, potassium hydroxide, magnesium hydroxide, calcium hydroxide, aluminum hydroxide, sodium carbonate, potassium carbonate, magnesium carbonate, calcium carbonate, sodium bicarbonate, potassium bicarbonate, disodium hydrogen phosphate, dipotassium hydrogen phosphate, trisodium phosphate, calcium monohydrogen phosphate, calcium phosphate, magnesium oxide, calcium oxide, magnesium silicate, calcium silicate, hydrotalcite, synthetic hydrotalcite, magnesium aluminate silicate, magnesium aluminate metasilicate, aluminum magnesium silicate, magnesium alumina hydroxide or a mixture thereof, and more preferably is magnesium hydroxide, calcium hydroxide, sodium carbonate, potassium carbonate, calcium carbonate, sodium bicarbonate, potassium bicarbonate, calcium monohydrogen phosphate, synthetic hydrotalcite or a mixture thereof, and still more preferably, magnesium hydroxide, calcium hydroxide, synthetic hydrotalcite or a mixture thereof, and particularly preferably magnesium hydroxide, synthetic hydrotalcite, or a mixture thereof. The mixture of the above-mentioned basic compounds may be a mixture of two or more kinds of arbitrary basic compounds.

The content of the basic substance which is a constituent ingredient of the medicinal composition of the present invention is not limited as long as it is a content which is pharmacologically and pharmaceutically acceptable, and for example, it can be 0.5 to 80 wt%, and particularly 1 to 60 wt%, and is 2 or 40 wt% more preferably.

In the present invention, the blending ratio of the triazole compound (I) and the basic substance is not limited as long as it is a blending ratio which is pharmacologically and pharmaceutically acceptable and, for example, it may be 1:0.01 to 1:400, and preferably is 1:0.1 to 1:0.4.

The medicinal composition of the present invention may contain an antioxidant and such an antioxidant can be, for example, a sulfite such as sodium hydrogen sulfite, sodium sulfite and sodium pyrosulfite; an edetate such as calcium disodium edetate, sodium edetate and tetrasodium edetate; an ascorbic acid such as ascorbic acid, sodium L-ascorbate, calcium ascorbate, L-ascorbic acid stearic acid ester and palmitic acid ascorbate; an erythorbic acid such as erythorbic acid and sodium erythorbate; a tocopherol such as natural vitamin E, dl-α-tocopherol, d-δ-tocopherol, dl-α-tocopherol acetate, dl-α-tocopherol succinate and d-α-tocopherol calcium succinate; dibutylhydroxytoluene; butylhydroxyanisole; propyl gallate or a mixture thereof, and preferably, ascorbic acid, sodium L-ascorbate, calcium ascorbate, L-ascorbic acid stearic acid ester, palmitic acid ascorbate, natural vitamin E, dl-α-tocopherol, d-δ-tocopherol, dl-α-tocopherol acetate, dl-α-tocopherol succinate, d-α-tocopherol calcium succinate or a mixture thereof, and more preferably, ascorbic acid, sodium L-ascorbate, dl-α-tocopherol, dl-α-tocopherol acetate, dl-α-tocopherol succinate, d-α-tocopherol calcium succinate or a mixture thereof. The mixture of the above-mentioned antioxidant may be a mixture of two or more kinds of arbitrary antioxidants.

When the medicinal composition of the present invention contains an antioxidant, there is no limitation to the content of the antioxidant in the medicinal composition as long as it is pharmacologically and pharmaceutically acceptable and, for example, it may be 0.01 to 10 wt%, and particularly 0.01 to 5 wt%, and more preferably 0.1 to 1 wt%.

In the present invention, the blending ratio of the triazole compound (I) and the antioxidant is not limited as long as it is a blending ratio which is pharmacologically and pharmaceutically acceptable and, for example, it may be 1: 0.00025 to 1:50, and preferably is 1:0.01 to 1:0.1.

In the present invention, a preferred blending ratio of the triazole compound (I), the basic substance and the antioxidant is 1:0.1:0.025.

The medicinal composition of the present invention can be in a pharmacologically and pharmaceutically acceptable form such as a tablet, capsule, granule, pill, powder, liquid, syrup, troche, suspension, emulsion, injection, suppository, ointment or patch.

The medicinal composition of the present invention may suitably contain pharmaceutically acceptable excipients and these excipients may be, for example, diluents, binders, disintegrants, lubricants, coating agents, flavouring/sweetening agents, solvents, solubilizers, suspending agents, viscosifying agents, emulsifiers, preservatives or isotonicity agents.

The diluent may be, for example, an organic diluent or an inorganic diluent, and the organic diluent may be, for example, a sugar derivative such as lactose, sucrose, glucose, mannitol, D-mannitol, L-mannitol and sorbitol; a starch derivative such as corn starch, potato starch, alphanized starch, partially alphanized starch, sodium carboxymethylstarch, dextrin and pullulan; a cellulose derivative such as crystalline cellulose, methylcellulose, hydroxypropyl cellulose, a low-substituted hydroxypropyl cellulose, hydroxypropyl methyl cellulose, carmellose, sodium carmellose, calcium carmellose and crosscarmellose sodium; a natural polymer compound such as gum arabic, alginic acid, sodium alginacte, gelatin and dextran; a synthetic polymer compound such as carboxyvinyl polymer, sodium polyacrylate, popidon and cross popidon; or a mixture thereof, and the inorganic diluent may be, for example, a silicate such as hydrated silicon dioxide, anhydrous silicic acid, and light anhydrous silicic acid; a sulfate such as calcium sulfate and magnesium sulfate; or a mixture thereof. The diluent is preferably an organic diluent, and more preferably, a sugar derivative, a cellulose derivative, or a mixture thereof, and still more preferably lactose, D-mannitol, a low-substituted hydroxypropyl cellulose, hydroxypropyl cellulose, or a mixture thereof.

The binder may be, for example, a starch derivative as mentioned in the above diluents; a cellulose derivative as mentioned in the above diluents; a natural polymer compound as mentioned in the above diluents; a synthetic polymer compound as mentioned in the above diluents; or a mixture thereof and preferably a cellulose derivative or a mixture thereof, and more preferably hydroxypropyl cellulose.

The disintegrant may be, for example, a starch derivative as mentioned in the above diluents; a cellulose derivative as mentioned in the above diluents; a natural polymer compound as mentioned in the above diluents; a synthetic polymer compound as mentioned in the above diluents; or a mixture thereof and preferably a cellulose derivative or a mixture thereof, and more preferably a low-substituted hydroxypropyl cellulose.

The lubricant may be, for example, talc; stearic acid; a stearic acid metal salt such as calcium stearate and magnesium stearate; a lauryl sulfate such as sodium lauryl sulfate and lauryl magnesium sulfate; a wax such as white beeswax and Camauba wax; a sugar derivative as mentioned in the above diluents; a starch derivative as mentioned in the above diluents; a silicate as mentioned in the above diluents; a hydrogenated plant oil; a sugar fatty acid ester; or a mixture thereof and preferably a stearic acid metal salt, a silicate or a mixture thereof, and more preferably calcium stearate, magnesium stearate, anhydrous silicic acid or a mixture thereof, and still more preferably magnesium stearate.

The coating agent may be, for example, a sugar derivative as mentioned in the above diluents; a starch derivative as mentioned in the above diluents; shellac; talc; a film forming agent such as hydroxypropyl cellulose, hydroxypropylmethyl cellulose, polyvinyl acetal diethylamino acetate, hydroxypropylmethyl cellulose phthalate, cellulose acetate phthalate, hydroxypropylmethyl cellulose acetate succinate, carboxymethylethyl cellulose, meta-acrylic acid copolymer and ethyl cellulose; a light blocking agent such as titanium oxide; a plasticizer such as polyethylene glycol, propylene glycol and triacetin; a colorant such as yellow iron sesquioxide and iron sesquioxide; or a mixture thereof and preferably talc, a film forming agent, a light blocking agent, a colorant or a mixture thereof and more preferably talc, hydroxypropylmethyl cellulose, titanium oxide, yellow iron sesquioxide, iron sesquioxide or a mixture thereof. Coating may be performed, for example, using a coating liquid which has a coating agent among these dissolved or dispersed in water.

The flavouring/sweetening agent may be a sweetener, acidifier or flavor usually used, for example.

The solvent may be, for example, purified water; vegetable oil such as sesame oil, soybean oil, corn oil, cotton seed oil, olive oil, peanut oil; oleic acid ethyl ester; myristic acid isopropyl ester; benzyl benzoate; or a mixture thereof, and preferably it is purified water, sesame oil or soybean oil, and is more preferably purified water.

The solubilizer may be, for example, ethanol; propylene glycol; polyethylene glycol; polysorbate; polyoxy ethylene hydrogenated castor oil such as polyoxy ethylene hydrogenated castor oil 60, or a mixture thereof, and preferably it is ethanol, propylene glycol or polyethylene glycol, and more preferably ethanol.

The suspending agent and viscosifying agent may be, for example, a cellulose derivative as mentioned in the above diluents; a natural polymer compound as mentioned in the above diluents; a synthetic polymer compound as mentioned in the above diluents; a colloidal clay such as bentonite and veegum; or a mixture thereof, and preferably it is a cellulose derivative, a natural polymer compound or a synthetic polymer compound, more preferably a cellulose derivative, and still more preferably hydroxypropylmethyl cellulose.

The emulsifier may be, for example, a polysorbate as mentioned in the above solubilizers; a polyoxy ethylene hydrogenated castor oil as mentioned in the above solubilizers; a colloidal clay as mentioned in the above suspending agents and viscosifying agents; an anionic surfactant such as sodium lauryl sulfate and calcium stearate; a cationic surfactant such as benzalkonium chloride; a polyoxyethylene alkyl ether; polyoxyethylene polyoxypropylene glycol; polyoxyethylene sorbitan fatty acid ester; sucrose fatty acid ester; a polyoxyl stearate; a lecithin such as soybean lecithin and egg yolk lecithin; or a mixture thereof, and preferably it is a polysorbate, polyoxyethylene hydrogenated castor oil, lecithin, or a mixture thereof, and more preferably polyoxyethylene hydrogenated castor oil.

The preservative may be, for example, benzoic acid; a benzoic acid salt such as sodium benzoate; a p-hydroxybenzoic acid ester such as methylparaben, propylparaben; an alcohol such as chlorobutanol, benzyl alcohol and phenylethyl alcohol; benzalkonium chloride; a phenol such as phenol and cresol; thimerosal; dehydroacetic acid; sorbic acid; or a mixture thereof, and preferably it is benzoic acid salt, a *p*-hydroxybenzoic acid ester or a mixture thereof, and more preferably sodium benzoate, methylparaben, propyl paraben, or a mixture thereof.

The isotonicity agent may be, for example, sodium chloride, glucose, or a mixture thereof.

Although the type and content of the above-mentioned excipients used may vary depending on the drug form such as a tablet and a capsule, it can be selected according to well-known technology in the field of pharmacy. For example, in the case of a tablet, the content of the binder, disintegrant, lubricant and coating agent in the medicinal composition is, usually 0.5 to 10 wt% (preferably 1 to 5 wt%), 1 to 50 wt% (preferably 5 to 40 wt%), 0.1 to 10 wt% (preferably 0.5 to 3 wt%), and 0.1 to 10 wt% (preferably 1 to 8 wt%), respectively.

The medicinal preparation of the present invention has an excellent antifungal effect on genus Candida, genus Aspergillus, Cryptococcus neoformans, or genus Trichophyton, and since it has excellent solubility, oral absorbency, preservation and handling stability (particularly preservation stability) and low toxicity, it is useful for a medicinal composition for warm-blooded animals (particularly for human beings) [particularly as a therapeutic and a prevention agent (preferably therapeutic agent) of fungal diseases].

In the present invention, fungal diseases may be a deep-seated mycosis, a deep-seated cutaneous mycosis, a superficial mycosis, etc.

The medicinal composition of the present invention has an excellent antifungal effect on fungi of genus Candida, genus Aspergillus, genus Cryptococcus, genus Mucor, genus Histoplasma, genus Blastomyces, genus Coccidioides, genus Paracoccidioides, genus Trichophyton, genus Epidermophyton, genus Microsporum, genus Malassezia, genus Pseudallesheria, genus Sporothrix, genus Rhinosporidium, genus Fonsecaea, genus Wangiella, genus Phialophora, genus Exophiala, genus Cladosporium, genus Alternaria, genus Aureobasidium, genus Chaetomium, genus Curvularia, genus Drechslera, genus Mycocentrospora, genus Phoma, genus Hendersonula, genus Scytalidium, genus Corynespora, genus Leptosphaeria, genus Madurella, genus Neotestudina, genus Scedosporium, genus Pyrenochaeta, genus Geotrichum, genus Trichosporon, genus Chrysosporium, genus Coprinus, genus Schizophyllum, genus Pneumocystis, genus Conidiobolus, genus Bacidiobolus, genus Paecilomyces, genus Penicillium, genus Acremonium, genus Fusarium, genus Scopulariopsis, genus Saccharomyces, genus Cephalosporium, genus Loboa, genus Rhizopus, genus Rhizomucor or genus Absidia, etc.

The present invention is
(1) a medicinal composition comprising a triazole compound (I), or a pharmaceutically acceptable ester thereof or a pharmaceutically acceptable salt thereof, and a pharmaceutically acceptable basic substance, or
(2) the medicinal composition according to (1) comprising a pharmaceutically acceptable antioxidant.

The present invention is preferably the medicinal composition according to (1) or (2):
(3) wherein Ar¹ is a phenyl group having from 1 to 3 substituents (wherein each of the substituents represents a halogen atom or a trifluoromethyl group);
(4) wherein Ar¹ is a phenyl group having 1 or 2 substituents (wherein each of the substituents represents a fluorine atom, a chlorine atom or a trifluoromethyl group);
(5) wherein Ar² represents a phenyl group, a 5- or 6-membered aromatic heterocyclic group (wherein the aromatic heterocyclic group has at least one nitrogen, oxygen or sulfur atom) or a phenyl or 5- or 6-membered aromatic heterocyclic group having from 1 to 3 substituents {wherein each of the substituents represents a lower alkyl group, a halogen atom, a lower alkyl group substituted with a halogen atom, a lower alkoxy group substituted with a halogen atom, a nitro group, a cyano group, or an -S(O)ₘR⁶ group (wherein R⁶ represents a lower alkyl group which may be substituted with a halogen atom, and m represents 0, 1 or 2) and the aromatic heterocyclic group has at least one nitrogen, oxygen or sulfur atom};
(6) wherein Ar² represents a phenyl group, a 5- or 6-membered aromatic heterocyclic group (wherein the aromatic heterocyclic group has one nitrogen, oxygen or sulfur atom) or a phenyl or 5- or 6-membered aromatic heterocyclic group having from 1 to 3 substituents {wherein each of the substituents represents a lower alkyl group, a halogen atom, a lower alkyl group substituted with a halogen atom, a lower alkoxy group substituted with a halogen atom, a nitro group, a cyano group or an -S(O)ₘR⁶ group (wherein R⁶ represents a lower alkyl group which may be substituted with a halogen atom and m represents 0, 1 or 2) and the aromatic heterocyclic group has one nitrogen, oxygen or sulfur atom};
(7) wherein Ar² represents a phenyl group, a 5- or 6-membered aromatic heterocyclic group (wherein the aromatic heterocyclic group has one nitrogen, oxygen or sulfur atom) or a phenyl or 5- or 6-membered aromatic heterocyclic group having 1 or 2 substituents {wherein each of the substituents represents a lower alkyl group, a halogen atom, a lower alkyl group substituted with a halogen atom, a lower alkoxy group substituted with a halogen atom, a nitro group, a cyano group or an - S(O)ₘR⁶ group (wherein R⁶ represents a lower alkyl group which may be substituted with a halogen atom and m represents 0, 1 or 2) and the aromatic heterocyclic group has one nitrogen or sulfur atom};
(8) wherein Ar² represents a phenyl group, a pyridyl group or a phenyl, pyridyl or thienyl group having 1 or 2 substituents {wherein each of the substituents represents a lower alkyl group, a halogen atom, a lower alkyl group substituted with a halogen atom, a lower alkoxy group substituted with a halogen atom, a nitro group, a cyano group or an -S(O)ₘR⁶ group (wherein R⁶ represents a lower alkyl group which may be substituted with a halogen atom and m represents 0, 1 or 2)};
(9) wherein R⁰ is a hydrogen atom, a methyl group, an ethyl group or a propyl group;
(10) wherein R⁰ is a hydrogen atom, a methyl group or an ethyl group;
(11) wherein R⁰ is a hydrogen atom or a methyl group;
(12) wherein R¹ is a methyl group, an ethyl group or a propyl group;
(13) wherein R¹ is a methyl group or an ethyl group;
(14) wherein R¹ is a methyl group;
(15) wherein R², R ³, R⁴ and R⁵ are the same or different and each represents a hydrogen atom, a lower alkyl group or a lower alkyl group substituted with a fluorine or a chlorine atom;
(16) wherein R², R ³, R⁴ and R⁵ are the same or different and each represents a hydrogen atom, a methyl group, an ethyl group, a propyl group, or a methyl, ethyl or propyl group substituted with a fluorine or chlorine atom;
(17) wherein R², R³, R⁴ and R⁵ are the same or different and each represents a hydrogen atom, a methyl group, an ethyl group, or a methyl or ethyl group substituted with a fluorine or chlorine atom; or
(18) wherein R², R ³, R⁴ and R⁵ are the same or different and each represents a hydrogen atom, a methyl group or a methyl group substituted with a fluorine atom.
   The medicinal composition wherein the triazole compound (I) is a compound obtained by arbitrarily combining Ar¹ selected from the above (3) or (4), Ar² selected from the above (5) to (8), R⁰ selected from the above (9) to (11), R¹ selected from the above (12) to (14), and R², R³, R⁴ and R⁵ selected from the above (15) to (18) is more preferred.
   For example, the medicinal composition of the above (1) or (2) is still more preferably such a medicinal composition
(19) wherein the triazole compound (I) is (2R,3R)-3-[[trans-2-[(1E,3E)-4-(4-cyano-2-fluorophenyl)-1,3-butadien-1-yl]-1, 3-dioxan-5-yl]thio]-2-(2,4-difluorophenyl)-1-(1H-1,2,4-triazol-1-yl)-2-butanol.

In the present invention, the medicinal composition of the above (1) or (2) is preferably such a medicinal composition
(20) wherein the basic substance is an alkaline metal hydroxide, an alkaline earth metal hydroxide, aluminum hydroxide, an alkaline metal carbonate, an alkaline earth metal carbonate, an alkaline metal bicarbonate, an alkaline metal dihydrogen phosphate, a dialkaline metal hydrogen phosphate, a trialkaline metal phosphate, an alkaline earth metal dihydrogen phosphate, an alkaline earth metal monohydrogen phosphate, an alkaline earth metal phosphate, an alkaline earth metal oxide, aluminum oxide, an alkaline metal silicate, an alkaline earth metal silicate, a complex silicate-aluminum compound, a complex aluminum-magnesium compound, a basic amino acid, a basic polymer, an amine or a mixture thereof;
(21) wherein the basic substance is an alkaline metal hydroxide, an alkaline earth metal hydroxide, aluminum hydroxide, an alkaline metal carbonate, an alkaline earth metal carbonate, an alkaline metal bicarbonate, a dialkaline metal hydrogen phosphate, a trialkaline metal phosphate, an alkaline earth metal monohydrogen phosphate, an alkaline earth metal phosphate, an alkaline earth metal oxide, an alkaline earth metal silicate, a complex aluminum-magnesium compound or a mixture thereof;
(22) wherein the basic substance is an alkaline earth metal hydroxide, a complex aluminum-magnesium compound or a mixture thereof;
(23) wherein the basic substance is lithium hydroxide, sodium hydroxide, potassium hydroxide, magnesium hydroxide, calcium hydroxide, barium hydroxide, aluminum hydroxide, lithium carbonate, sodium carbonate, potassium carbonate, magnesium carbonate, calcium carbonate, barium carbonate, lithium bicarbonate, sodium bicarbonate, potassium bicarbonate, lithium dihydrogen phosphate, sodium dihydrogen phosphate, potassium dihydrogen phosphate, dilithium hydrogen phosphate, disodium hydrogen phosphate, dipotassium hydrogen phosphate, trilithium phosphate, trisodium phosphate, tripotassium phosphate, magnesium dihydrogen phosphate, calcium dihydrogen phosphate, barium dihydrogen phosphate, magnesium monohydrogen phosphate, calcium monohydrogen phosphate, barium monohydrogen phosphate, magnesium phosphate, calcium phosphate, barium phosphate, barium oxide, magnesium oxide, calcium oxide, aluminum oxide, lithium silicate, sodium silicate, potassium silicate, barium silicate, magnesium silicate, calcium silicate, silicic acid-alumina, hydrotalcite, synthetic hydrotalcite, magnesium aluminate silicate, magnesium aluminate metasilicate, aluminum magnesium silicate, magnesium alumina hydroxide, L-arginine, L-lysine, an aminoalkyl methacrylate copolymer, monoethanolamine, diethanolamine, diisopropanolamine, triethanolamine, triisopropanolamine, ethylenediamine or a mixture thereof;
(24) wherein the basic substance is sodium hydroxide, potassium hydroxide, magnesium hydroxide, calcium hydroxide, aluminum hydroxide, sodium carbonate, potassium carbonate, magnesium carbonate, calcium carbonate, sodium bicarbonate, potassium bicarbonate, disodium hydrogen phosphate, dipotassium hydrogen phosphate, trisodium phosphate, calcium monohydrogen phosphate, calcium phosphate, magnesium oxide, calcium oxide, magnesium silicate, calcium silicate, hydrotalcite, synthetic hydrotalcite, magnesium aluminate silicate, magnesium aluminate metasilicate, aluminum magnesium silicate, magnesium alumina hydroxide or a mixture thereof;
(25) wherein the basic substance is magnesium hydroxide, calcium hydroxide, sodium carbonate, potassium carbonate, calcium carbonate, sodium bicarbonate, potassium bicarbonate, calcium monohydrogen phosphate, synthetic hydrotalcite or a mixture thereof;
(26) wherein the basic substance is magnesium hydroxide, calcium hydroxide, synthetic hydrotalcite or a mixture thereof; or
(27) wherein the basic substance is magnesium hydroxide, synthetic hydrotalcite or a mixture thereof.

A combination of a medicinal composition wherein the triazole compound (I) is a compound obtained by arbitrarily combining Ar¹ selected from the above (3) or (4), Ar² selected from the above (5) to (8), R° selected from the above (9) to (11), R¹ selected from the above (12) to (14), and R², R³, R⁴ and R⁵ selected from the above (15) to (18) [preferably the above-mentioned (19) compound] and the basic substance which is a constituent ingredient is a substance selected from the above (20) to (27) is more preferred.

Furthermore, the present invention is preferably the medicinal composition of the above (2),
(28) wherein the antioxidant is a sulfite, edetic acid, ascorbic acid, erythorbic acid, a tocopherol, dibutylhydroxytoluene, butylhydroxyanisole, propyl gallate or a mixture thereof;
(29) wherein the antioxidant is sodium hydrogen sulfite, sodium sulfite, sodium pyrosulfite, disodium calcium edetate, sodium edetate, tetrasodium edetate, ascorbic acid, sodium L-ascorbate, calcium ascorbate, L-ascorbic acid stearic acid ester, palmitic acid ascorbate, erythorbic acid, sodium erythorbate, natural vitamin E, dl-α-tocopherol, d-δ-tocopherol, dl-α-tocopherol acetate, dl-α-tocopherol succinate, d-α-tocopherol calcium succinate, dibutylhydroxytoluene, butylhydroxyanisol, propyl gallate or a mixture thereof;
(30) wherein the antioxidant is ascorbic acid, sodium L-ascorbate, calcium ascorbate, L-ascorbic acid stearic acid ester, palmitic acid ascorbate, natural vitamin E, dl-α-tocopherol, d-δ-tocopherol, dl-α-tocopherol acetate, dl-α-tocopherol succinate, d-α-tocopherol calcium succinate or a mixture thereof;
(31) wherein the antioxidant is ascorbic acid, sodium L-ascorbate, dl-α-tocopherol, dl-α-tocopherol acetate, d-α-tocopherol calcium succinate or a mixture thereof.

A combination of a medicinal composition wherein the triazole compound (I) is a compound obtained by arbitrarily combining Ar¹ selected from the above (3) or (4), Ar² selected from the above (5) to (8), R⁰ selected from the above (9) to (11), R¹ selected from the above (12) to (14), and R², R³, R⁴ and R⁵ selected from the above (15) to (18) [preferably the above-mentioned (19) compound] and the basic substance which is a constituent ingredient is a substance selected from the above (20) to (27) and when it has an antioxidant as a composition ingredient, the antioxidant is a substance selected from the above (28) to (31) is more preferred.

For example, the medicinal compositions of the above-described (1) to (31) are still preferably such medicinal compositions
(32) wherein the triazole compound (I) is (2R,3R)-3-[[trans-2-[(1E,3E)-4-(4-cyano-2-fluorophenyl)-1,3-butadien-1-yl]-1,3-dioxan-5-yl]thio]-2-(2,4-difluorophenyl)-1-(1H-1,2,4-triazol-1-yl)-2-butanol, and the basic substance is lithium hydroxide, sodium hydroxide, potassium hydroxide, magnesium hydroxide, calcium hydroxide, barium hydroxide, aluminum hydroxide, lithium carbonate, sodium carbonate, potassium carbonate, magnesium carbonate, calcium carbonate, barium carbonate, lithium bicarbonate, sodium bicarbonate, potassium bicarbonate, lithium dihydrogen phosphate, sodium dihydrogen phosphate, potassium dihydrogen phosphate, dilithium hydrogen phosphate, disodium hydrogen phosphate, dipotassium hydrogen phosphate, trilithium phosphate, trisodium phosphate, tripotassium phosphate, magnesium dihydrogen phosphate, calcium dihydrogen phosphate, barium dihydrogen phosphate, magnesium monohydrogen phosphate, calcium monohydrogen phosphate, barium monohydrogen phosphate, magnesium phosphate, calcium phosphate, barium phosphate, barium oxide, magnesium oxide, calcium oxide, aluminum oxide, lithium silicate, sodium silicate, potassium silicate, barium silicate, magnesium silicate, calcium silicate, silicic acid-alumina, hydrotalcite, synthetic hydrotalcite, magnesium aluminate silicate, magnesium aluminate metasilicate, aluminum magnesium silicate, magnesium alumina hydroxide, L-arginine, L-lysine, an aminoalkyl methacrylate copolymer, monoethanolamine, diethanolamine, diisopropanolamine, triethanolamine, triisopropanolamine, ethylenediamine or a mixture thereof; and still more preferably such medicinal compositions
(33) wherein the triazole compound (I) is (2R,3R)-3-[[trans-2-[(1E,3E)-4-(4-cyano-2-fluorophenyl)-1,3-butadien-1-yl]-1,3-dioxan-5-yl]thio]-2-(2,4-difluorophenyl)-1-(1H-1,2,4-triazol-1-yl)-2-butanol and the basic substance is sodium hydroxide, potassium hydroxide, magnesium hydroxide, calcium hydroxide, aluminum hydroxide, sodium carbonate, potassium carbonate, magnesium carbonate, calcium carbonate, sodium bicarbonate, potassium bicarbonate, disodium hydrogen phosphate, dipotassium hydrogen phosphate, trisodium phosphate, calcium monohydrogen phosphate, calcium phosphate, magnesium oxide, calcium oxide, magnesium silicate, calcium silicate, hydrotalcite, synthetic hydrotalcite, magnesium aluminate silicate, magnesium aluminate metasilicate, aluminum magnesium silicate, magnesium alumina hydroxide or a mixture thereof; and particularly preferably such medicinal compositions
(34) wherein the triazole compound (I) is (2R,3R)-3-[[trans-2-[(1E,3E)-4-(4-cyano-2-fluorophenyl)-1,3-butadien-1-yl]-1,3-dioxan-5-yl]thio]-2-(2,4-difluorophenyl)-1-(1H-1,2,4-triazol-1-yl)-2-butanol and the basic substance is magnesium hydroxide, calcium hydroxide, sodium carbonate, potassium carbonate, calcium carbonate, sodium bicarbonate, potassium bicarbonate, calcium monohydrogen phosphate, synthetic hydrotalcite or a mixture thereof; and most preferably such medicinal compositions
(35) wherein the triazole compound (I) is (2R,3R)-3-[[trans-2-[(1E,3E)-4-(4-cyano-2-fluorophenyl)-1,3-butadien-1-yl]-1,3-dioxan-5-yl]thio]-2-(2,4-difluorophenyl)-1-(1H-1,2,4-triazol-1-yl)-2-butanol and the basic substance is magnesium hydroxide, synthetic hydrotalcite or a mixture thereof.

Furthermore, the medicinal compositions of the above-described (2) to (31) are still more preferably such medicinal compositions,
(36) wherein the triazole compound (I) is (2R,3R)-3-[[trans-2-[(1E,3E)-4-(4-cyano-2-fluorophenyl)-1,3-butadien-1-yl]-1,3-dioxan-5-yl]thio]-2-(2,4-difluorophenyl)-1-(1H-1,2,4-triazol-1-yl)-2-butanol and the basic substance is lithium hydroxide, sodium hydroxide, potassium hydroxide, magnesium hydroxide, calcium hydroxide, barium hydroxide, aluminum hydroxide, lithium carbonate, sodium carbonate, potassium carbonate, magnesium carbonate, calcium carbonate, barium carbonate, lithium bicarbonate, sodium bicarbonate, potassium bicarbonate, lithium dihydrogen phosphate, sodium dihydrogen phosphate, potassium dihydrogen phosphate, dilithium hydrogen phosphate, disodium hydrogen phosphate, dipotassium hydrogen phosphate, trilithium phosphate, trisodium phosphate, tripotassium phosphate, magnesium dihydrogen phosphate, calcium dihydrogen phosphate, barium dihydrogen phosphate, magnesium monohydrogen phosphate, calcium monohydrogen phosphate, barium monohydrogen phosphate, magnesium phosphate, calcium phosphate, barium phosphate, barium oxide, magnesium oxide, calcium oxide, aluminum oxide, lithium silicate, sodium silicate, potassium silicate, barium silicate, magnesium silicate, calcium silicate, silicic acid-alumina, hydrotalcite, synthetic hydrotalcite, magnesium aluminate silicate, magnesium aluminate metasilicate, aluminum magnesium silicate, magnesium alumina hydroxide, L-arginine, L-lysine, an aminoalkyl methacrylate copolymer, monoethanolamine, diethanolamine, diisopropanolamine, triethanolamine, triisopropanolamine, ethylenediamine or a mixture thereof and the antioxidant is sodium hydrogen sulfite, sodium sulfite, sodium pyrosulfite, disodium calcium edetate, sodium edetate, tetrasodium edetate, ascorbic acid, sodium L-ascorbate, calcium ascorbate, L-ascorbic acid stearic acid ester, palmitic acid ascorbate, erythorbic acid, sodium erythorbate, natural vitamin E, dl-α-tocopherol, d-δ-tocopherol, dl-α-tocopherol acetate, dl-α-tocopherol succinate, d-α-tocopherol calcium succinate, dibutylhydroxytoluene, butylhydroxyanisol, propyl gallate or a mixture thereof; particularly preferably such medicinal compositions,
(37) wherein the triazole compound (I) is (2R,3R)-3-[[trans-2-[(1E,3E)-4-(4-cyano-2-fluorophenyl)-1,3-butadien-1-yl]-1,3-dioxan-5-yl]thio]-2-(2,4-difluorophenyl)-1-(1H-1,2,4-triazol-1-yl)-2-butanol and the basic substance is magnesium hydroxide, calcium hydroxide, sodium carbonate, potassium carbonate, calcium carbonate, sodium bicarbonate, potassium bicarbonate, calcium monohydrogen phosphate, synthetic hydrotalcite or a mixture thereof and the antioxidant is ascorbic acid, sodium L-ascorbate, calcium ascorbate, L-ascorbic acid stearic acid ester, palmitic acid ascorbate, natural vitamin E, dl-α-tocopherol, d-δ-tocopherol, dl-α-tocopherol acetate, dl-α-tocopherol succinate, d-α-tocopherol calcium succinate or a mixture thereof; and most preferably such medicinal compositions,
(38) wherein the triazole compounds (I) is (2R,3R)-3-[[trans-2-[(1E,3E)-4-(4-cyano-2-fluorophenyl)-1,3-butadien-1-yl]-1,3-dioxan-5-yl]thio]-2-(2,4-difluorophenyl)-1-(1H-1,2,4-triazol-1-yl)-2-butanol and the basic substance is magnesium hydroxide, synthetic hydrotalcite or a mixture thereof and the antioxidant is ascorbic acid, sodium L-ascorbate, dl-α-tocopherol, dl-α-tocopherol acetate, d-α-tocopherol calcium succinate or a mixture thereof.

Furthermore, the present invention is preferably
(39) the medicinal composition according to any of (1) to (38), wherein the medicinal composition is a therapeutic agent for fungal diseases;
(40) the medicinal composition according to any of (1) to (38), wherein the medicinal composition is a prophylactic agent for fungal diseases;
(41) a medicinal composition according to (39) or (40), wherein the medicinal composition is for warm-blooded animals; or
(42) a medicinal composition according to (39) or (40), wherein the medicinal composition is for human beings.

The triazole compound (I) or a pharmaceutically acceptable ester thereof or a pharmaceutically acceptable salt thereof, which is a constituent ingredient of the medicinal composition of the present invention, can be prepared easily according to a well-known method (for example, Japanese Patent No. 2902345, Japanese Patent No. 3240129) or a method similar thereto.

The medicinal composition of the present invention is easily prepared using the triazole compound (I), a pharmaceutically acceptable ester thereof or a pharmaceutically acceptable salt thereof, a pharmaceutically acceptable basic substance and excipients according to a well-known method such as a kneading method using water, a wet granulation method. For example, a diluent, a disintegrant and a basic substance are added to the triazole compound (I), a pharmaceutically acceptable ester thereof or a pharmaceutically acceptable salt thereof and mixed by a high-speed mixing and granulating machine, an aqueous solution of a binder is added to the obtained mixture and granulated, the obtained wet granules are dried using a fluidized bed drier, the size of the dried granules is reduced using a screening mill, a lubricant is added and mixed with a V-shaped blender, and a tablet or a capsule can be prepared by compressing the obtained mixture into a tablet or filling the obtained mixture into a capsule.

The obtained tablet can be subjected to sugar-coating or coating (preferably coating) if needed. For example, film coating can be performed by atomizing a coating liquid comprising hydroxypropylmethyl cellulose, talc, titanium oxide, yellow iron sesquioxide or iron sesquioxide, and water onto the obtained tablet in a pan coating machine.

Alternatively, the obtained blend is granulated using an extruding granulating machine to give granules and the granules are dried with a fence type drier and the dried granulated substance is passed under pressure through a screen using a pulverizing granulating machine and thereby granulates can be prepared.

The medicinal composition of the present invention can be administered to a warm-blooded animal (particularly a human being) and although the dose of the triazole compound (I), a pharmaceutically acceptable ester thereof or a pharmaceutically acceptable salt thereof which is a constituent ingredient may vary depending on various conditions such as the activity of each drug, condition, age and weight of the patient, it is desirable to administer 1 mg (preferably 5 mg) at the minimum and 2000 mg (preferably 1000 mg) at the maximum per time in the case of oral administration and 0.1 mg (preferably 0.5 mg) at the minimum and 600 mg (preferably 500 mg) at the maximum per time in the case of intravenous administration for a human adult 1 to 6 times per day according to the condition.

### Best Mode for Carrying Out the Invention

Although the present invention will be described in more detail hereafter by way of Example, Referential Example and Test Example, the range of the present invention is not limited thereto. (2R,3R)-3-[[trans-2-[(1E,3E)-4-(4-cyano-2-fluorophenyl)-1,3-butadien-1-yl]-1,3-dioxan-5-yl]thio]-2-(2,4-difluorophenyl)-1-(1H-1,2,4-triazol-1-yl)-2-butanol was used as the test compound.

### (Example 1) Tablet

A tablet containing the test compound was prepared by the following method using the kind and amount of ingredient shown in Table 1.

To the test compound, a diluent (lactose or D-mannitol), a disintegrant (low-substituted hydroxypropyl cellulose) and a basic substance (synthetic hydrotalcite or magnesium hydroxide) were added, mixed by a high-speed mixing and granulating machine, and a solution of a binder (hydroxypropyl cellulose) was added to the obtained mixture and granulated. The obtained wet granules were dried using an Air-through tray dryer, the size of the dried granules was reduced using a screening mill, and a lubricant (magnesium stearate) was added and mixed with a V-shaped blender. The obtained mixture was shaped using a pestle having a diameter of 9 mm, and 250 mg of tablet were obtained.

**Table 1**

| Ingredient | | Amount per one tablet (mg) | | | |
|---|---|---|---|---|---|
| | (Tablet No.) | 1 | 2 | 3 | 4 |
| Test compound | | 10 | 10 | 10 | 10 |
| Lactose | | 171.25 | 171.25 | | |
| D-mannitol | | | | 171.25 | 171.25 |
| Low-substituted hydroxypropyl cellulose | | 50 | 50 | 50 | 50 |
| Magnesium hydroxide | | 10 0 | | 10 | |
| Synthetic hydrotalcite | | | 10 | | 10 |
| Hydroxypropyl cellulose | | 7.5 | 7.5 | 7.5 | 7.5 |
| Magnesium stearate | | 1.25 | 1.25 | 1.25 | 1.25 |
| Total | | 250 | 250 | 250 | 250 |

### (Referential Example 1) Comparative Tablet

A comparative tablet containing the test compound was prepared by the following method using the kind and amount of ingredient shown in Table 2.

To the test compound, a diluent (lactose or D-mannitol) and a disintegrant (low-substituted hydroxypropyl cellulose) were added, mixed by a high-speed mixing and granulating machine, and a solution of a binder (hydroxypropyl cellulose) was added to the obtained mixture and granulated. The obtained wet granules were dried using an Air-through tray dryer, the size of the dried granules was reduced using a screening mill, and a lubricant (magnesium stearate) was added and mixed with a V-shaped blender. The obtained mixture was shaped using a pestle having a diameter of 9 mm, and 250 mg of tablet were obtained.

**Table 2**

| Ingredient | | Amount per one tablet (mg) | |
|---|---|---|---|
| | (Tablet No.) | 5 | 6 |
| Test compound | | 10 | 10 |
| Lactose | | 181.25 | |
| D-mannitol | | | 181.25 |
| Low-substituted hydroxypropyl cellulose | | 50 | 50 |
| Hydroxypropyl cellulose | | 7.5 | 7.5 |
| Magnesium stearate | | 1.25 | 1.25 |
| Total | | 250 | 250 |

### (Test Example 1) Stability Test

The test tablets obtained in Example and comparative tablets obtained in Referential Example were put into brown glass bottles, allowed to stand still at 60°C in a sealed state, and impurities in the tablets after three weeks were measured using high-speed liquid chromatography. The conditions for measurement of the high-speed liquid chromatography are as follows.
Column:
Develosil ODS-MG-5 (4.6 mm ID x 25 cm, manufactured by Nomura Kagaku)
Column temperature: 40°C
Mobile phase A: acetonitrile/water mixture (3/2)
Mobile phase B: acetonitrile/water mixture (3/1)
Flow rate: about 1 mL per minute
Gradient condition:
For 25 minutes after starting injection, the mobile phase A at 100% was sent and for the next 15 minutes (from 25 minutes to 40 minutes after starting injection) the ratio was changed in a linear gradient so that the ratio of the mobile phase B might go from 0 to 100%, and for the next 30 minutes (from 40 minutes to 70 minutes after starting injection), the mobile phase B at 100% was sent.

Detection wavelength: 220 nm

The peak area derived from impurities was determined from the obtained chromatogram, and the area ratio to the peak area of the test compound was calculated. The sum total (%) of the impurities in the tablet using lactose as a diluent is shown in Table 3 and the sum total (%) of the impurities in the tablet using D-mannitol as a diluent is shown in Table 4.

**Table 3**

| Total impurities (%) | | | |
|---|---|---|---|
| (Tablet No.) | At start of stability test | In 3 weeks at 60°C | Increase |
| 1 | 2.09 | 2.91 | 0.82 |
| 2 | 1.95 | 2.53 | 0.58 |
| 5 | 2.21 | 3.63 | 1.42 |

**Table 4**

| Total impurities (%) | | | |
|---|---|---|---|
| (Tablet No.) | At start of stability test | In 3 weeks at 60°C | Increase |
| 3 | 2.30 | 2.81 | 0.51 |
| 4 | 2.17 | 2.61 | 0.44 |
| 6 | 2.35 | 3.25 | 0.90 |

As shown in Table 3 and 4, since the medicinal composition containing a basic substance of the present invention has an excellent preservation stability as compared with a medicinal composition which does not contain a basic substance, it is useful as a medicinal preparation.

### Industrial Applicability

Since the medicinal composition of the present invention has an excellent preservation and handling stability (particularly preservation stability), it is useful as a medicinal preparation for warm-blooded animals (particularly for human beings) [particularly as a therapeutic and a prevention agent (preferably therapeutic agent) for fungal diseases].

## Claims

1. A medicinal composition comprising a triazole compound having the general formula (I): 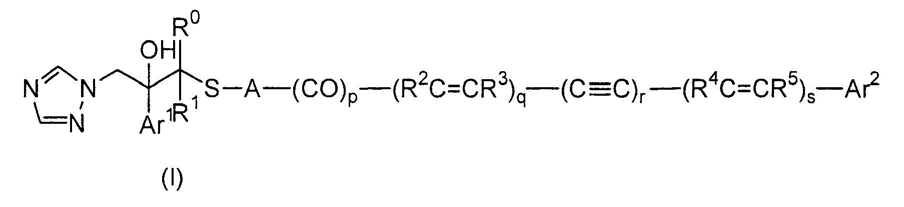 [wherein Ar¹ represents a phenyl group or a phenyl group having from 1 to 3 substituents (wherein each of the substituents represents a halogen atom or a trifluoromethyl group);
Ar² represents a phenyl group, a 5- or 6-membered aromatic heterocyclic group (wherein the aromatic heterocyclic group has at least one nitrogen, oxygen or sulfur atom) or a phenyl or 5- or 6-membered aromatic heterocyclic group having from 1 to 3 substituents {wherein each of the substituents represents a lower alkyl group, a lower alkoxy group, a halogen atom, a lower alkyl group substituted with a halogen atom, a lower alkoxy group substituted with a halogen atom, a nitro group, a cyano group, an -S(O)ₘR⁶ group (wherein R⁶ represents a lower alkyl group which may be substituted with a halogen atom and m represents 0, 1 or 2) or an NHCOR⁷ group (wherein R⁷ represents a lower alkyl group) and the aromatic heterocyclic group has at least one nitrogen, oxygen or sulfur atom};
R⁰represents a hydrogen atom or a lower alkyl group;
R¹ represents a lower alkyl group;
R², R³, R⁴ and R⁵ are the same or different and each represents a hydrogen atom, a lower alkyl group or a lower alkyl group substituted with a halogen atom and R², R³, R⁴ and R⁵ each independently represents the same or different group when q and/or s represents 2;
p represents 0 or 1;
q, r and s each represent 0, 1 or 2; and
A represents 1,3-dioxane],
or a pharmaceutically acceptable ester thereof or a pharmaceutically acceptable salt thereof, and
a pharmaceutically acceptable basic substance.

2. The medicinal composition according to claim 1 further comprising a pharmaceutically acceptable antioxidant.

3. The medicinal composition according to claim 1 or 2, wherein the triazole compound is (2R,3R)-3-[[trans-2-[(1E,3E)-4-(4-cyano-2-fluorophenyl)-1,3-butadien-1 -yl] -1,3-dioxan-5-yl]thio]-2-(2,4-difluorophenyl)-1 -(1 1 H-1,2,4-triazol-1-yl)-2-butanol.

4. The medicinal composition according to any one selected from claims 1 to 3, wherein the basic substance is an alkaline metal hydroxide, an alkaline earth metal hydroxide, aluminum hydroxide, an alkaline metal carbonate, an alkaline earth metal carbonate, an alkaline metal bicarbonate, an alkaline metal dihydrogen phosphate, a dialkaline metal hydrogen phosphate, a trialkaline metal phosphate, an alkaline earth metal dihydrogen phosphate, an alkaline earth metal monohydrogen phosphate, an alkaline earth metal phosphate, an alkaline earth metal oxide, aluminum oxide, an alkaline metal silicate, an alkaline earth metal silicate, a complex silicate-aluminum compound, a complex aluminum-magnesium compound, a basic amino acid, a basic polymer, an amine or a mixture thereof.

5. The medicinal composition according to any one selected from claims 1 to 3, wherein the basic substance is an alkaline metal hydroxide, an alkaline earth metal hydroxide, aluminum hydroxide, an alkaline metal carbonate, an alkaline earth metal carbonate, an alkaline metal bicarbonate, a dialkaline metal hydrogen phosphate, a trialkaline metal phosphate, an alkaline earth metal monohydrogen phosphate, an alkaline earth metal phosphate, an alkaline earth metal oxide, an alkaline earth metal silicate, a complex aluminum-magnesium compound or a mixture thereof.

6. The medicinal composition according to any one selected from claims 1 to 3, wherein the basic substance is an alkaline earth metal hydroxide, a complex aluminum-magnesium compound or a mixture thereof.

7. The medicinal composition according to any one selected from claims 1 to 3, wherein the basic substance is lithium hydroxide, sodium hydroxide, potassium hydroxide, magnesium hydroxide, calcium hydroxide, barium hydroxide, aluminum hydroxide, lithium carbonate, sodium carbonate, potassium carbonate, magnesium carbonate, calcium carbonate, barium carbonate, lithium bicarbonate, sodium bicarbonate, potassium bicarbonate, lithium dihydrogen phosphate, sodium dihydrogen phosphate, potassium dihydrogen phosphate, dilithium hydrogen phosphate, disodium hydrogen phosphate, dipotassium hydrogen phosphate, trilithium phosphate, trisodium phosphate, tripotassium phosphate, magnesium dihydrogen phosphate, calcium dihydrogen phosphate, barium dihydrogen phosphate, magnesium monohydrogen phosphate, calcium monohydrogen phosphate, barium monohydrogen phosphate, magnesium phosphate, calcium phosphate, barium phosphate, barium oxide, magnesium oxide, calcium oxide, aluminum oxide, lithium silicate, sodium silicate, potassium silicate, barium silicate, magnesium silicate, calcium silicate, silicic acid-alumina, hydrotalcite, synthetic hydrotalcite, magnesium aluminate silicate, magnesium aluminate metasilicate, aluminum magnesium silicate, magnesium alumina hydroxide, L-arginine, L-lysine, an aminoalkyl methacrylate copolymer, monoethanolamine, diethanolamine, diisopropanolamine, triethanolamine, triisopropanolamine, ethylenediamine or a mixture thereof.

8. The medicinal composition according to any one selected from claims 1 to 3, wherein the basic substance is sodium hydroxide, potassium hydroxide, magnesium hydroxide, calcium hydroxide, aluminum hydroxide, sodium carbonate, potassium carbonate, magnesium carbonate, calcium carbonate, sodium bicarbonate, potassium bicarbonate, disodium hydrogen phosphate, dipotassium hydrogen phosphate, trisodium phosphate, calcium monohydrogen phosphate, calcium phosphate, magnesium oxide, calcium oxide, magnesium silicate, calcium silicate, hydrotalcite, synthetic hydrotalcite, magnesium aluminate silicate, magnesium aluminate metasilicate, aluminum magnesium silicate, magnesium alumina hydroxide or a mixture thereof.

9. The medicinal composition according to any one selected from claims 1 to 3, wherein the basic substance is magnesium hydroxide, calcium hydroxide, sodium carbonate, potassium carbonate, calcium carbonate, sodium bicarbonate, potassium bicarbonate, calcium monohydrogen phosphate, synthetic hydrotalcite or a mixture thereof.

10. The medicinal composition according to any one selected from claims 1 to 3, wherein the basic substance is magnesium hydroxide, calcium hydroxide, synthetic hydrotalcite or a mixture thereof.

11. The medicinal composition according to any one selected from claims 1 to 3, wherein the basic substance is magnesium hydroxide, synthetic hydrotalcite or a mixture thereof.

12. The medicinal composition according to any one selected from claims 2 to 11, wherein the antioxidant is a sulfite, edetic acid, ascorbic acid, erythorbic acid, a tocopherol, dibutylhydroxytoluene, butylhydroxyanisole, propyl gallate or a mixture thereof.

13. The medicinal composition according to any one selected from claims 2 to 11, wherein the antioxidant is sodium hydrogen sulfite, sodium sulfite, sodium pyrosulfite, disodium calcium edetate, sodium edetate, tetrasodium edetate, ascorbic acid, sodium L-ascorbate, calcium ascorbate, L-ascorbic acid stearic acid ester, palmitic acid ascorbate, erythorbic acid, sodium erythorbate, natural vitamin E, dl-α-tocopherol, d-δ-tocopherol, dl-α-tocopherol acetate, dl-α-tocopherol succinate, d-α-tocopherol calcium succinate, dibutylhydroxytoluene, butylhydroxyanisol, propyl gallate or a mixture thereof.

14. The medicinal composition according to any one selected from claims 2 to 11, wherein the antioxidant is ascorbic acid, sodium L-ascorbate, calcium ascorbate, L-ascorbic acid stearic acid ester, palmitic acid ascorbate, natural vitamin E, dl-α-tocopherol, d-δ-tocopherol, dl-α-tocopherol acetate, dl-α-tocopherol succinate, d-α-tocopherol calcium succinate or a mixture thereof.

15. The medicinal composition according to any one selected from claims 2 to 11, wherein the antioxidant is ascorbic acid, sodium L-ascorbate, dl-α-tocopherol, dl-α-tocopherol acetate, d-α-tocopherol calcium succinate or a mixture thereof.

16. The medicinal composition according to any one selected from claims 1 to 15, wherein the medicinal composition is a therapeutic agent for fungal diseases.

17. The medicinal composition according to any one selected from claims 1 to 15, wherein the medicinal composition is a prophylactic agent for fungal diseases.

18. The medicinal composition according to claim 16 or 17, wherein the medicinal composition is for warm-blooded animals.

19. The medicinal composition according to claim 16 or 17, wherein the medicinal composition is for humans.
